# EUROPEAN PATENT SPECIFICATION

(11) **EP 1 311 267 B1**
(45) Date of publication and mention of the grant of the patent: **20.06.2012**
(21) Application number: 01962453.5
(22) Date of filing: 16.08.2001
(51) Int. Cl.: A61K 31/475, A61K 39/21, A61P 31/18, A61P 37/02

(54) **A RETROVIRAL IMMUNOTHERAPY**
RETROVIRALE IMMUNTHERAPIE
IMMUNOTHERAPIE ANTI-RETROVIRALE

(30) Priority: 18.08.2000 AU PQ048800
(43) Date of publication of application: 21.05.2003
(73) Proprietor: Immunaid Pty Ltd, Fitzroy, Victoria 3065 (AU)
(72) Inventor: ASHDOWN, Martin, Leonard, Thornbury, Victoria 3071 (AU)
(74) Representative: Harding, Charles Thomas
(86) International application number: PCT/AU2001/001019
(87) International publication number: WO 2002/013828

(56) References cited:
- DE-A1- 4 120 296
- GHEE T. TAN ET AL.: "Evaluation of natural products as inhibitors of human immunodeficiency virus type 1 (HIV-1) reverse transcriptase" JOURNAL OF NATURAL PRODUCTS, vol. 54, no. 1, 1991, pages 143-154, XP002450461
- DATABASE MEDLINE [Online] DEZUBE B.J., XP002964469 Database accession no. 10950369 & SEMIN. ONCOL. vol. 27, no. 4, August 2000, pages 424 - 430
- DATABASE MEDLINE [Online] AVERSA S.M. ET AL., XP002964470 Database accession no. 10228499 & TUMORI vol. 85, no. 1, January 1999 - February 1999, pages 54 - 59
- DATABASE MEDLINE [Online] ERRANTE D. ET AL., XP002964471 Database accession no. 10093688 & ANN. ONCOL. vol. 10, no. 2, February 1999, pages 189 - 195
- DATABASE MEDLINE [Online] NASTI G. ET AL., XP002964472 Database accession no. PMID:10348092 & DRUG SAF. vol. 20, no. 5, May 1999, pages 403 - 425
- DATABASE MEDLINE [Online] ZANUSSI S. ET AL., XP002964473 Database accession no. 8959247 & AIDS RES. HUM. RETROVIRUSES vol. 12, no. 18, 10 December 1996, pages 1703 - 1707
- DATABASE MEDLINE [Online] NORTH R.J. ET AL., XP002964474 Database accession no. 2145216 & IMMUNOLOGY vol. 71, no. 1, September 1990, pages 90 - 95
- LORI F ET AL: "Targeting HIV reservoirs and reconstituting the immune system", AIDS RESEARCH AND HUMAN RETROVIRUSES 1999 US LNKD- DOI:10.1089/088922299309649, vol. 15, no. 18, 1999, pages 1597-1617, ISSN: 0889-2229
- LORI F: "Hydroxyurea and HIV: 5 years later - From antiviral to immune-modulating effects", AIDS 1999 GB LNKD- DOI:10.1097/00002030-199908200-00001, vol. 13, no. 12, 1999, pages 1433-1442, ISSN: 0269-9370
- DAAR E S ET AL: "Acute HIV syndrome after discontinuation of antiretroviral therapy in a patient treated before seroconversion", ANNALS OF INTERNAL MEDICINE, NEW YORK, NY; US, US, vol. 128, no. 10, 15 May 1998 (1998-05-15) , pages 827-829, XP002488509, ISSN: 0003-4819
- MANFRED W BEILHARZ ET AL: "Timed Ablation of Regulatory CD4 T Cells Can Prevent Murine AIDS Progression", JOURNAL OF IMMUNOLOGY, AMERICAN ASSOCIATION OF IMMUNOLOGISTS, US, vol. 172, no. 8, 15 April 2004 (2004-04-15), pages 4917-4925, XP007905048, ISSN: 0022-1767
- BERZOFSKY J A ET AL: "APPROACHES TO IMPROVE ENGINEERED VACCINES FOR HUMAN IMMUNODEFICIENCY VIRUS AND OTHER VIRUSES THAT CAUSE CHRONIC INFECTIONS", IMMUNOLOGICAL REVIEWS, BLACKWELL PUBLISHING, MUNKSGAARD, vol. 170, 1 August 1999 (1999-08-01), pages 151-172, XP008029856, ISSN: 0105-2896, DOI: 10.1111/J.1600-065X.1999.TB01336.X
- MWAU M ET AL: "A review of vaccines for HIV prevention", JOURNAL OF GENE MEDICINE 200301 GB LNKD- DOI:10.1002/JGM.343, vol. 5, no. 1, January 2003 (2003-01), pages 3-10, ISSN: 1099-498X
- LIU Z ET AL: "Epitope-vaccine strategy against HIV-1: today and tomorrow", IMMUNOBIOLOGY, URBAN UND FISCHER VERLAG, DE, vol. 208, no. 4, 1 January 2003 (2003-01-01), pages 423-428, XP004954164, ISSN: 0171-2985, DOI: 10.1078/0171-2985-00286
- SAKAGUCHI S ET AL: "Regulatory T Cells: Key Controllers of Immunologic Self-Tolerance", CELL, CELL PRESS, US, vol. 101, 26 May 2000 (2000-05-26), pages 455-458, XP003025450, ISSN: 0092-8674, DOI: 10.1016/S0092-8674(00)80856-9
- READ S ET AL: "CD4<+> regulatory T cells", CURRENT OPINION IN IMMUNOLOGY, ELSEVIER, OXFORD, GB, vol. 13, no. 6, 1 December 2001 (2001-12-01), pages 644-649, XP004311242, ISSN: 0952-7915, DOI: 10.1016/S0952-7915(01)00273-4
- BEYER MARC ET AL: "Regulatory T cells in cancer", BLOOD, AMERICAN SOCIETY OF HEMATOLOGY, US, vol. 108, no. 3, 1 August 2006 (2006-08-01), pages 804-811, XP002549092, ISSN: 0006-4971, DOI: 10.1182/BLOOD-2006-02-002774 [retrieved on 2006-04-06]
- NORTH R J ET AL: "Elimination of cycling CD4+ suppressor T cells with an anti-mitotic drug releases non-cycling CD8+ T cells to cause regression of an advanced lymphoma", IMMUNOLOGY, BLACKWELL PUBLISHING, OXFORD, GB, vol. 71, no. 1, 1 September 1990 (1990-09-01), pages 90-95, XP002488512, ISSN: 0019-2805
- BIRGIT KNOECHEL ET AL: 'Sequential development of interleukin 2-dependent effector and regulatory T cells in response to endogenous systemic antigen' JOURNAL OF EXPERIMENTAL MEDICINE vol. 202, no. 10, 21 November 2005, pages 1375 - 1386, XP055001304 ISSN: 0022-1007
- HIURA T ET AL: "Both regulatory T cells and antitumor effector T cells are primed in the same draining lymph nodes during tumor progression", JOURNAL OF IMMUNOLOGY 20051015 US, vol. 175, no. 8, 15 October 2005 (2005-10-15), pages 5058-5066, ISSN: 0022-1767
- WEISS L ET AL: "Human immunodeficiency virus-driven expansion of CD4<+>CD25 <+> regulatory T cells, which suppress HIV-specific CD4 T-cell responses in HIV-infected patients", BLOOD 20041115 US LNKD- DOI:10.1182/BLOOD-2004-01-0365, vol. 104, no. 10, 15 November 2004 (2004-11-15), pages 3249-3256, ISSN: 0006-4971
- KINTER AUDREY L ET AL: "CD25+ CD4+ regulatory T cells from the peripheral blood of asymptomatic HIV-infected individuals regulate CD4+ and CD8+ HIV-specific T cell immune responses in vitro and are associated with favorable clinical markers of disease status", JOURNAL OF EXPERIMENTAL MEDICINE, vol. 200, no. 3, 2 August 2004 (2004-08-02), pages 331-343, ISSN: 0022-1007
- LIM A Y -F ET AL: "Cell surface markers of regulatory T cells are not associated with increased forkhead box p3 expression in blood CD4<+> T cells from HIV-infected patients responding to antiretroviral therapy", IMMUNOLOGY AND CELL BIOLOGY 200612 AU LNKD- DOI:10.1111/J.1440-1711.2006.01467.X, vol. 84, no. 6, December 2006 (2006-12), pages 530-536, ISSN: 0818-9641
- NILSSON JAKOB ET AL: "HIV-1-driven regulatory T-cell accumulation in lymphoid tissues is associated with disease progression in HIV/AIDS", BLOOD, AMERICAN SOCIETY OF HEMATOLOGY, US, vol. 108, no. 12, 1 December 2006 (2006-12-01), pages 3808-3817, XP002480781, ISSN: 0006-4971, DOI: 10.1182/BLOOD-2006-05-021576 [retrieved on 2006-08-10]
- KINTER A ET AL: "Suppression of HIV-specific T cell activity by lymph node CD25<+> regulatory T cells from HIV-infected individuals", PROCEEDINGS OF THE NATIONAL ACADEMY OF SCIENCES OF THE UNITED STATES OF AMERICA 20070227 US LNKD- DOI:10.1073/PNAS.0611423104, vol. 104, no. 9, 27 February 2007 (2007-02-27), pages 3390-3395, ISSN: 0027-8424
- KINTER A L ET AL: "CD25<+> regulatory T cells isolated from HIV-infected individuals suppress the cytolytic and nonlytic antiviral activity of HIV-specific CD8 <+> T cells in vitro", AIDS RESEARCH AND HUMAN RETROVIRUSES 200703 US LNKD- DOI:10.1089/AID.2006.0162, vol. 23, no. 3, March 2007 (2007-03), pages 438-450, ISSN: 0889-2229
- LIM A ET AL: "Proportions of circulating T cells with a regulatory cell phenotype increase with HIV-associated immune activation and remain high on antiretroviral therapy", AIDS 200707 GB LNKD- DOI:10.1097/QAD.0B013E32825EAB8B, vol. 21, no. 12, July 2007 (2007-07), pages 1525-1534, ISSN: 0269-9370
- CAO W ET AL: "Regulatory T cell expansion and immune activation during untreated HIV type 1 infection are associated with disease progression", AIDS RESEARCH AND HUMAN RETROVIRUSES 20090201 US LNKD- DOI:10.1089/AID.2008.0140, vol. 25, no. 2, 1 February 2009 (2009-02-01), pages 183-191, ISSN: 0889-2229
- VAHLENKAMP ET AL: "The role of CD4<+>CD25<+> regulatory T cells in viral infections", VETERINARY IMMUNOLOGY AND IMMUNOPATHOLOGY, ELSEVIER BV, AMSTERDAM, NL, vol. 108, no. 1-2, 18 October 2005 (2005-10-18), pages 219-225, XP005077466, ISSN: 0165-2427, DOI: 10.1016/J.VETIMM.2005.07.011
- BELKAID Y ET AL: "Natural regulatory T cells in infectious disease", NATURE IMMUNOLOGY 2005 GB LNKD- DOI:10.1038/NI1181, vol. 6, no. 4, 2005, pages 353-360, ISSN: 1529-2908
- ROUSE B T ET AL: "Regulatory T cells in virus infections", IMMUNOLOGICAL REVIEWS 200608 GB LNKD- DOI:10.1111/J.0105-2896.2006.00412.X, vol. 212, August 2006 (2006-08), pages 272-286, ISSN: 0105-2896
- DITTMER U ET AL: "Functional impairment of CD8<+> T cells by regulatory T cells during persistent retroviral infection", IMMUNITY 200403 US LNKD- DOI:10.1016/S1074-7613(04)00054-8, vol. 20, no. 3, March 2004 (2004-03), pages 293-303, ISSN: 1074-7613
- IWASHIRO M ET AL: "Immunosuppression by CD4+ regulatory T cells induced by chronic retroviral infection", PROCEEDINGS OF THE NATIONAL ACADEMY OF SCIENCES, NATIONAL ACADEMY OF SCIENCES, WASHINGTON, DC; US, vol. 98, no. 16, 31 July 2001 (2001-07-31) , pages 9226-9230, XP002488511, ISSN: 0027-8424, DOI: 10.1073/PNAS.151174198
- VAHLENKAMP THOMAS W ET AL: "Feline immunodeficiency virus infection phenotypically and functionally activates immunosuppressive CD4+CD25+ T regulatory cells", JOURNAL OF IMMUNOLOGY, vol. 172, no. 8, 15 April 2004 (2004-04-15), pages 4752-4761, ISSN: 0022-1767
- ESTES J D ET AL: "Simian immunodeficiency virus-induced lymphatic tissue fibrosis is mediated by transforming growth factor [beta]1-positive regulatory T cells and begins in early infection", JOURNAL OF INFECTIOUS DISEASES 20070215 US LNKD- DOI:10.1086/510852, vol. 195, no. 4, 15 February 2007 (2007-02-15), pages 551-561, ISSN: 0022-1899
- HRYNIEWICZ A ET AL: "CTLA-4 blockade decreases TGF-[beta], IDO, and viral RNA expression in tissues of SIVmac251-infected macaques", BLOOD 20061201 US LNKD- DOI:10.1182/BLOOD-2006-04-010637, vol. 108, no. 12, 1 December 2006 (2006-12-01), pages 3834-3842, ISSN: 0006-4971

## Description

### FIELD OF THE INVENTION:

The present invention provides a method of treating a retroviral infection in a mammalian subject. More particularly, the present invention provides a method of treating a retroviral infection which leads to an immunodeficiency-related disease in a human subject.

### BACKGROUND OF THE INVENTION:

Human immunodeficiency virus (HIV) induces a persistent and progressive infection leading, in the vast majority of cases, to the development of the acquired immunodeficiency syndrome (AIDS). There are at least two distinct types of HIV: HIV-1 and HIV-2. In humans, HIV infection eventually leads to immune incompetence, opportunistic infections, neurological dysfunctions, neoplastic growth, and ultimately death.

HIV is a member of the lentivirus family of retroviruses. Retroviruses are small enveloped viruses that contain a single-stranded RNA genome, and replicate via the insertion of a DNA intermediate into the host DNA produced by a virally-encoded reverse transcriptase. Other retroviruses include, for example, oncogenic viruses such as human T-cell leukemia viruses (HTLV-I,-II,-III), feline leukemia virus, and the murine type C retroviruses.

The HIV viral particle consists of a viral core, composed in part of capsid proteins designated p24 and p18, together with the viral RNA genome and those enzymes required for early replicative events. Myristylated gag protein forms an outer viral shell around the viral core, which is, in turn, surrounded by a lipid membrane envelope derived from the infected cell membrane. The HIV envelope surface glycoproteins are synthesized as a single 160 kilodalton precursor protein which is cleaved by a cellular protease during viral budding into two glycoproteins, gp41 and gp120. gp41 is a transmembrane glycoprotein and gp120 is an extracellular glycoprotein which remains non-covalently associated with gyp41, possibly in a trimeric or multimeric form.

HIV infection is pandemic and HIV-associated diseases represent a major world health problem. Although considerable effort is being put into the design of effective therapeutics, currently no curative anti-retroviral drugs or therapies against AIDS exist. In attempts to develop such drugs, several stages of the HIV life cycle have been considered as targets for therapeutic intervention (Mitsuya et al., 1991).

Attention has been given to the development of vaccines for the treatment of HIV infection. The HIV-1 envelope proteins (gp160, gp120, gp41) have been shown to be the major antigens for anti-HIV antibodies present in AIDS patients (Barin et al., 1985). Thus far, therefore, these proteins seem to be the most promising candidates to act as antigens for anti-HIV vaccine development. Several groups have begun to use various portions of gp160, gp120, and/or gp41 as immunogenic targets for the host immune system (US 5,141,867; WO 92/22654; WO 91/09872; WO 90/07119; US 6,090,392). Despite these efforts, an effective vaccine strategy for the treatment of HIV infection has not been developed.

The present invention provides an alternate immunotherapy for treating a retroviral infection.

### SUMMARY OF THE INVENTION:

The present inventor has noted that at least two populations of immune cells are produced in response to retroviruses which infect mammals. More particularly, the immune system of a mammal infected with a retrovirus is capable of mounting an immune response against the virus through a group of cells herein generally referred to as "effector cells", however, a second population of cells are also produced which regulate the "effector cells", herein generally referred to as "regulator cells", limiting the mammal's ability to effectively control or eradicate the retroviral infection.

Whilst not wishing to be limited by theory, it is proposed that humans contain many sequences which are homologous or near homologous to retroviral sequences fragmented throughout their genome as stable heritable elements. Accordingly, proteins encoded by these sequences may be recognised as "self' during development of the immune response. Subsequent infection by a retrovirus may then also be partially recognized as "self", limiting the immune system's ability to mount a successful immune response. This proposal, at least in part, may explain why up until now it has been difficult to develop an effective vaccine against HIV.

Support for this hypothesis has been provided by Rakowicz-Szulczynska and co-workers (1998 and 2000) who have shown that some antigens associated with breast cancer are molecularly and immunologically similar to proteins encoded by HIV-1. Similar observations have been made for other cancers. In addition, Coll et al. (1995) have reported antibodies which bind HIV-1 in patients with autoimmune diseases such as Sjogren's syndrome and systemic lupus erythematosus. Furthermore, a BLAST search of the human genome database with the human immunodeficiency virus genome sequences indicates many regions of significant identity between the two genomes.

It has also been noted that the relative number of effector cells expand in response to an antigen before the regulator cells expand. This provides an opportunity to prevent the production of, or destroy, the "regulator cells" whilst maintaining the "effector cells".

Accordingly, the present invention provides uses and a method as defined in the appended claims. In a first aspect disclosed herein is a method of treating a retroviral infection in a mammalian subject, the method comprising administering to the subject a composition which increases the number of, and/or activates, effector cells directed against the retrovirus, and subsequently administering to the subject an agent which inhibits the production of, and/or destroys, regulator cells, wherein the timing of administration of the agent is selected such that the activity of the effector cells is not significantly reduced.

There are many ways in which the number, and/or activity, of effector cells directed against a retrovirus can be increased. In some instances, this will occur by an inadvertent infection with the retrovirus, for example, a needle prick of a syringe containing the virus. As is well known, health workers and researchers run the risk of viral infection through needle stick injury. Similarly, the general public are also exposed to this danger through discarded syringes, particularly on the beach, and muggings with such syringes. Therefore, upon suspected exposure to a retrovirus, particularly HIV, the mammalian subject could utilize the method of the present invention.

The method can also be used to treat a mammalian subject which has been infected with a retrovirus for some time. Although the immune system of such a subject has already been exposed to the retrovirus, the addition of further retroviral antigens may lead to a further effector cell response with subsequent opportunity to ablate the regulators of these new effectors.

Subjects infected with a retrovirus can be treated with antiretroviral drugs to keep the viral load low. Such subjects also could be administered with the composition to initiate a new effector cell immune response whilst the subsequent administration of the agent would ablate regulator cells.

In a preferred embodiment of the first aspect, the agent is administered approximately when CD8+CD4- T cell numbers have peaked in response to the administration of the composition.

Further, it is preferred that the agent is administered approximately when the number of viral particles has begun to stabilize or increase following administration of the composition.

In yet another preferred embodiment of the first aspect, restimulation of effector cells in an infected mammalian subject can be achieved by a composition which comprises a retroviral antigenic polypeptide. Preferably, the antigenic polypeptide is provided to the subject by administering a vaccine comprising the retrovirus antigenic polypeptide and a pharmaceutically acceptable carrier. More preferably, the vaccine further comprises an adjuvant

In another embodiment the antigenic polypeptide is provided to the subject by administering a DNA vaccine encoding the retroviral antigenic polypeptide.

In yet another embodiment, the antigenic polypeptide is provided to the subject by the consumption of a transgenic plant expressing the retroviral antigenic polypeptide.

Withdrawal of antiretroviral treatment typically causes a rapid re-expansion of effector cells against the re-emergent virus in an infected subject. Accordingly, at the appropriate time the agent can be administered to ablate the regulator cells without the need to administer a composition as defined herein.

Therefore, in a second aspect disclosed herein is a method of treating a retroviral infection in a mammalian subject, the method comprising exposing the subject to antiretroviral drug therapy, and subsequently administering to the subject an agent which inhibits the production of, and/or destroys, regulator cells, wherein the agent is administered after the antiretroviral drug therapy has concluded and a resulting expansion in retroviral numbers has led to an increase in the number, and/or activation, of effector cells directed against the retrovirus, and wherein the timing of administration of the agent is selected such that the activity of effector cells is not significantly reduced.

In a preferred embodiment of the second aspect, the agent is administered approximately when CD8+CD4- T cell numbers have peaked in response to the conclusion of the antiretroviral drug therapy.

Upon removal of antiretroviral drug therapy the viral load increases (for example, see Daar et al., 1998). This results in an expansion and/or activation of effector cells directed against the retrovirus which begin to stabilize the viral load. It is approximately when the retroviral numbers have peaked, or begun to decrease after this peak, that the agent should be administered.

Accordingly, in a further preferred embodiment of the second aspect, the agent is administered approximately when the number of viral particles has peaked, or begun to decrease after this peak, in response to the conclusion of the antiretroviral drug therapy.

Preferably, the agent used in regulator cell ablation is selected from the group consisting of anti-proliferative drugs, radiation, and an antibody which binds specifically to CD4+ T cells. Preferably, the anti-proliferative drug is selected from the group consisting of vinblastine and anhydro vinblastine.

Preferably, the retrovirus is selected from the group consisting of H1V-1, HIV-2, HTLV-1 and HTLV-2.

As would be readily appreciated by those skilled in the art, the methods may be repeated to provide a more complete treatment.

Preferably, the mammalian subject is a human.

In a third aspect, disclosed herein is the use of an agent which inhibits the production of, and/or destroys, regulator cells in the manufacture of a medicament for treatment of a retroviral infection in a mammalian subject.

In a preferred embodiment of the third aspect, the agent is selected from the group consisting of anti-proliferative drugs, and an antibody which binds specifically to CD4+ T cells.

In each aspect outlined above, ablation of the "regulator" population allows the "effector" population to reduce or eradicate the retroviral load as any down regulation of effectors (due to self-tolerance mechanisms) has been removed.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention will hereinafter be described by way of the following non-limiting Figures and Examples.

### BRIEF DESCRIPTION OF THE DRAWINGS:

Figure 1: Time course of MAIDS in B6 mice infected with LP-BM5.
Figure 2: Effect of a single dose of vinblastine (6mg/kg i.p.) on MAIDS progression at 10 weeks post infection.
Figure 3: Spleen histology of vinblastine treated mice 10 weeks post infection.
Figure 4: Protection from MAIDS at 20 weeks post infection following vinblastine therapy. n=5.
Figure 5: Rechallenge with MAIDS virus following protective vinblastine therapy. n=5.
Figure 6: Effect of day 14 vinblastine on spleen cell percentages in MAIDS infected (MAIDS+) and control mice (MAIDS-). n=3.
Figure 7: Spleen transfer experimental protocol.
Figure 8: Spleen cell transfers from MAIDS infected donor mice. n=7.
Figure 9: *In vivo* depletion experimental protocol.
Figure 10. *In vivo* depletion of CD4+ or CD8+ cells at day 14 post infection. n=5.

### DETAILED DESCRIPTION OF THE INVENTION:

### Immune Response to Retroviral Infection

As used herein the term "treating" or "treat" means a reduction in retroviral load is achieved. Most preferably, the retroviral load is completely eradicated.

Although the precise nature of the "regulator cells" has not yet been determined it has been established that these cells include a subpopulation of CD4+ cells.

CD4+ cells express the marker known in the art as CD4. Typically, the term "CD4+ T cells" as used herein does not refer to cells which also express CD8. However, this term can include T cells which also express other antigenic markers such as CD25.

Although the precise nature of the "effector cells" has not yet been determined, it is known that these cells include the T cell population known as CD8+ cells.

As used herein, the term "ablate" or "ablation" when referring to the exposure of the "regulator cells" to the agent means that the number, and/or activity, of regulator cells is down-regulated by the agent. Most preferably, the number, and/or activity, of regulator cells is completely eradicated by the agent.

### Agents which Inhibit the Production of, and/or Destroy, Regulator Cells

The agent can be any factor or treatment which selectively or non-selectively results in the destruction, or the inhibition of the production, of regulator cells. For example, a CD₄+ specific antibody could be used to specifically target CD4+ T cells. However, in some instances a non-selective agent could be used, such as an anti-proliferative drug or radiation, both of which destroy dividing cells.

The term "anti-proliferative drug" is a term well understood in the art and refers to any compound that destroys dividing cells or inhibits them from undergoing further proliferation. Anti-proliferative drugs include, mechlorethamine, cyclophosphamide, ifosfamide, melphalan, chlorambucil, hexamethyl-melamine, thiotepa, busulfan, carmustine, lomustine, semustine, streptozocin, dacarbazine, methotrexate, fluorouracil, floxuridine, cytarabine, mercaptopurine, thioguanine, pentostatin, vinblastine, anhydro vinblastine, vincristine, etoposide, teniposide, dactinomycin, daunorubicin, doxorubicin, bleomycin, plicamycin, mitomycin, L-asparaginase, cisplatin, mitoxantrone, hydroxyurea, procarbazine, mitotane, aminoglutethimide, prednisone, hydroxyprogesterone caproate, medroprogesterone acetate, megestrol acetate, diethylstilbestrol, ethinyl estradiol, tamoxifen, testosterone propionate, radioactive isotopes, ricin A chain, taxol, diphtheria toxin and pseudomonas exotoxin A.

### Timing of Exposing the Subject to the Agent

As outlined above, the present invention relies on the observation that the relative number of effector cells expands in response to an antigen before the regulator cells. Accordingly, as used herein, the term "the activity of the effector cells is not significantly reduced" means that the timing of the administration of the agent is such that the agent exerts a proportionally greater effect against the regulator cells than the effector cells. It is clearly preferred that the agent is administered at a time where the ratio of effect against the regulator cells to the effect against effector cells is greatest.

It has been reported that after the initial fall in viral load following anti-retroviral treatment, for instance in subjects infected with HIV, there is an increase in viral load and a subsequent stimulation of the immune response to the increase in viral load upon withdrawal of treatment (Oritz et al., 1999; Kilby et al., 2000; Lifson et al., 2000). Accordingly, the exposure of the subject to anti-viral therapy followed by removal of the therapy could be used to increase the number of, and/or activate, effector cells directed against the retrovirus enabling a targetable regulator cell expansion.

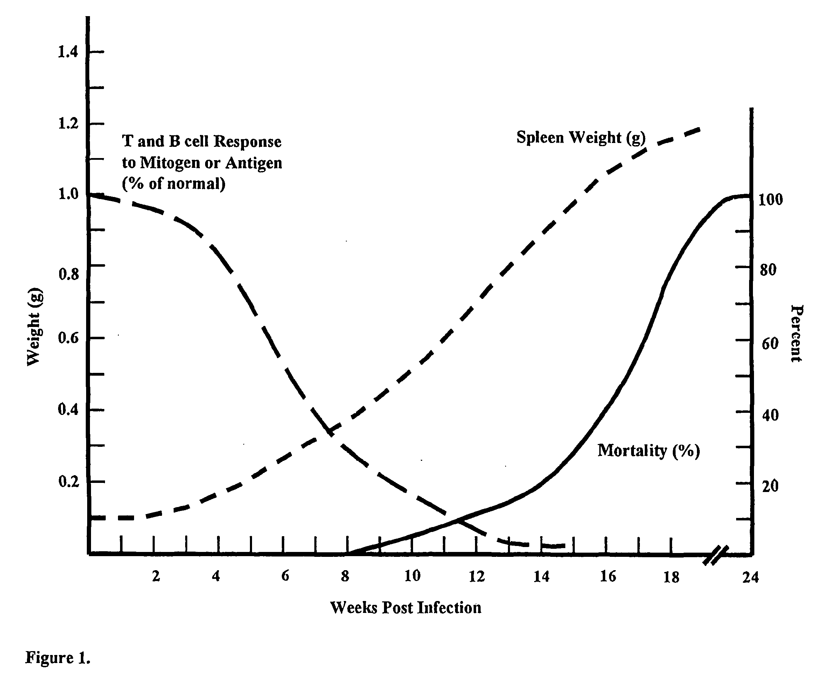

An example of the appropriate time for administering the agent can be determined by reference to Daar et al. (1998). This document provides the viral load, and CD8+ and CD₄+ T cell levels, from a patient who is taken off highly active antiretroviral therapy (also known as HEART). After the initial rise in viral load, a reduction in viral load (shoulder at day ~225) occurs when the CD8+ T cells have reached maximum numbers, and therefore effect (see Figure 1 of Daar et al., 1998). Immediately beyond this time point (a few days) CD8+ T cells start to drop off and viral load begins to stabilize after the decline. This point (the peaks) could be used as an inference point to predict the clonal expansion of the subsequent regulator cells and therefore an intervention point for regulator cell ablation. It is where the viral load stops declining that the regulator cells have down regulated the effector cells, but it is before this point that the agent should be applied, namely when the majority of regulators are in clonal expansion (mitosis).

Techniques known in the art can be used to monitor the growing population of regulator cells following the stimulation of an immune response.

Serial blood samples can be collected and quantitatively screened for all CD4+ subsets by FACS analysis. This FACS monitoring will need to be maintained until the regulator cells begin clonally expanding in response to the stimulus. In most instances, this time point will need to be empirically determined in subjects at different stages of infection as their immune response kinetics may vary. Other possible assays include lymphocyte proliferation/activation assays and various cytokine level assays (for example an assay for IL-6).

Another avenue of determining the time point for administering the agent is to monitor the viral load following stimulation of the immune response. It is envisaged that the viral load decreases due to the activity of the effector cells, however, the subsequent increase in regulator cells would down-regulate the effector cells resulting in a slowing of the viral load decrease. Accordingly, the agent could be administered prior to the slowing of the decrease in viral load. Techniques known in the art, for example RT-PCR, could be used to monitor viral load in these circumstances.

Monitoring may need to be very frequent, for example every few hours, to ensure the correct time point is selected for administration of the agent.

Optimally, the monitoring is continued to determine the affect of the agent Insufficient ablation, re-emergence of the regulator cells or increases in viral load within, for example, about 7 days of treatment will mean that the method should be repeated. Such repeated cycles of treatment may generate immunological memory. It is therefore possible that the present invention, used in repetitive mode, may provide some prophylactic protective effect

### Antigenic Polypeptides

As used herein, an "antigenic" polypeptide is any polypeptide sequence that contains an epitope which is capable of producing an immune response against the retrovirus. Typically, the antigenic polypeptide will comprise a sequence which is highly conserved in most retroviral isolates. However, it is envisaged that a particular retrovirus infecting an individual could be characterized and an antigenic polypeptide produced which matches the sequences of the isolate to maximise the possibility of an effective immune response.

Information regarding HIV antigens such as gp120 and other candidates can be found in Stott et al (1998).

The antigenic polypeptides can be provided in any manner known in the art which leads to an immune response. Antigenic polypeptides can be, for example, native, recombinant or synthetic. Such antigenic polypeptides include, but are not limited to, viral proteins that are responsible for attachment to cell surface receptors to initiate the infection process such as envelope glycoproteins.

Native antigenic polypeptides can be prepared, for example, by providing attenuated retrovirus, heat inactivated retrovirus or any other killed retrovirus.

The antigenic polypeptides can be provided as isolated polypeptides in a vaccine composition. In this instance the polypeptide can be purified from retroviral infected cells, expressed and isolated from recombinant cells, or synthetically produced using a peptide synthesizer.

Unless otherwise indicated, the recombinant DNA techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (Editors), Current Protocols in Molecular Biology, Greene Pub. Associates and Wiley-Interscience (1988, including all updates until present).

### Vaccines

Vaccines may be prepared from one or more retroviral polypeptides. The preparation of vaccines which contain an antigenic polypeptide is known to one skilled in the art. Typically, such vaccines are prepared as injectables, or orals, either as liquid solutions or suspensions; solid forms suitable for solution in, or suspension in, liquid prior to injection or oral consumption may also be prepared. The preparation may also be emulsified, or the protein encapsulated in liposomes. The antigenic polypeptides are often mixed with carriers/excipients which are pharmaceutically acceptable and compatible with the active ingredient. Suitable carriers/excipients are, for example, water, saline, dextrose, glycerol, ethanol, or the like and combinations thereof.

In addition, if desired, the vaccine may contain minor amounts of auxiliary substances such as wetting or emulsifying agents, pH buffering agents, and/or adjuvants which enhance the effectiveness of the vaccine.

As used herein, the term "adjuvant" means a substance that non-specifically enhances the immune response to an antigenic polypeptide. Examples of adjuvants which may be effective include but are not limited to: aluminum hydroxide, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion. Further examples of adjuvants include aluminum hydroxide, aluminum phosphate, aluminum potassium sulfate (alum), bacterial endotoxin, lipid X, *Corynebacterium parvum (Propionobacterium acnes), Bordetella pertussis,* polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, N.J.) or Freund's Incomplete Adjuvant and Complete Adjuvant (Difco Laboratories, Detroit, Michigan).

The proportion of antigenic polypeptide and adjuvant can be varied over a broad range so long as both are present in effective amounts. For example, aluminum hydroxide can be present in an amount of about 0.5% of the vaccine mixture (Al₂O₃ basis). Conveniently, the vaccines are formulated to contain a final concentration of antigenic polypeptide in the range of from 0.2 to 200 µg/ml, preferably 5 to 50 µg/ml, most preferably 15 µg/ml.

After formulation, the vaccine may be incorporated into a sterile container which is then sealed and stored at a low temperature, for example 4°C, or it may be freeze-dried. Lyophilisation permits long-term storage in a stabilised form.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the vaccine composition is lyophilised, the lyophilised material may be reconstituted prior to administration, e.g. as a suspension. Reconstitution is preferably effected in buffer.

Capsules, tablets and pills for oral administration to a patient may be provided with an enteric coating comprising, for example, Eudragit "S", Eudragit "L", cellulose acetate, cellulose acetate phthalate or hydroxypropylmethyl cellulose.

### DNA Vaccination

DNA vaccination involves the direct *in vivo* introduction of DNA encoding an antigen into tissues of a subject for expression of the antigen by the cells of the subject's tissue. Such vaccines are termed herein "DNA vaccines" or "nucleic acid-based vaccines". DNA vaccines are described in US 5,939,400, US 6,110,898, WO 95/20660 and WO 93/19183.

The ability of directly injected DNA that encodes an antigen to elicit a protective immune response has been demonstrated in numerous experimental systems (see, for example, Conry et al., 1994; Cardoso *et al.,* 1996; Cox et al., 1993; Davis et al., 1993; Sedegah et al., 1994; Montgomery et al., 1993; Ulmer et al., 1993; Wang et al., 1993; Xiang et al., 1994; Yang et al., 1997).

To date, most DNA vaccines in mammalian systems have relied upon viral promoters derived from cytomegalovirus (CMV). These have had good efficiency in both muscle and skin inoculation in a number of mammalian species. A factor known to affect the immune response elicited by DNA immunization is the method of DNA delivery, for example, parenteral routes can yield low rates of gene transfer and produce considerable variability of gene expression (Montgomery et al., 1993). High-velocity inoculation of plasmids, using a gene-gun, enhanced the immune responses of mice (Fynan et al., 1993; Eisenbraun et al., 1993), presumably because of a greater efficiency of DNA transfection and more effective antigen presentation by dendritic cells. Vectors containing the nucleic acid-based vaccine may also be introduced into the desired host by other methods known in the art, e.g., transfection, electroporation, microinjection, transduction, cell fusion, DEAE dextran, calcium phosphate precipitation, lipofection (lysosome fusion), or a DNA vector transporter.

### Vaccines Derived from Transgenic Plants

Transgenic plants producing a retroviral antigenic polypeptide can be constructed using procedures well known in the art A number of plant-derived edible vaccines are currently being developed for both animal and human pathogens (Hood and Jilka, 1999). Immune responses have also resulted from oral immunization with transgenic plants producing virus-like particles (VLPs), or chimeric plant viruses displaying antigenic epitopes (Mason *et al.*, 1996; Modelska *et al.*, 1998; Kapustra *et al.*, 1999; Brennan *et al.*, 1999). It has been suggested that the particulate form of these VLPs or chimeric viruses may result in greater stability of the antigen in the stomach, effectively increasing the amount of antigen available for uptake in the gut (Mason *et al.* 1996, Modelska *et al.* 1998).

### EXAMPLES

In order to demonstrate the present invention a murine AIDS model was used.

A murine AIDS (MAIDS) pathology induced by LP-BM5 murine leukemia virus (MuLV) in susceptible mice is an effective tool to investigate mechanisms of retrovirus-induced immunodeficiency. The MAIDS animal model displays a number of features of human AIDS. Infection of a susceptible strain such as C57BL/6 mice with LP-BM5 leads to chronic splenomegaly, hypergammaglobulinaemia and development of immunodeficiency in both T and B cells. *In vitro*, there is a progressive impairment in the responsiveness of T-cells and B-cells to mitogenic or specific antigenic stimuli. *In vivo*, infected mice become increasingly susceptible to challenge with a variety of opportunistic organisms and can develop B-cell lymphoma. Deaths are first observed at 8-10 weeks post-infection (pi), and all mice die within 24 weeks (Figure 1). These alterations in immune function reflect complex changes in the phenotype and function of all components of the immune network.

### 1. Therapeutic Effects of a Single Dose of Vinblastine Administered at Different Times Post Infection

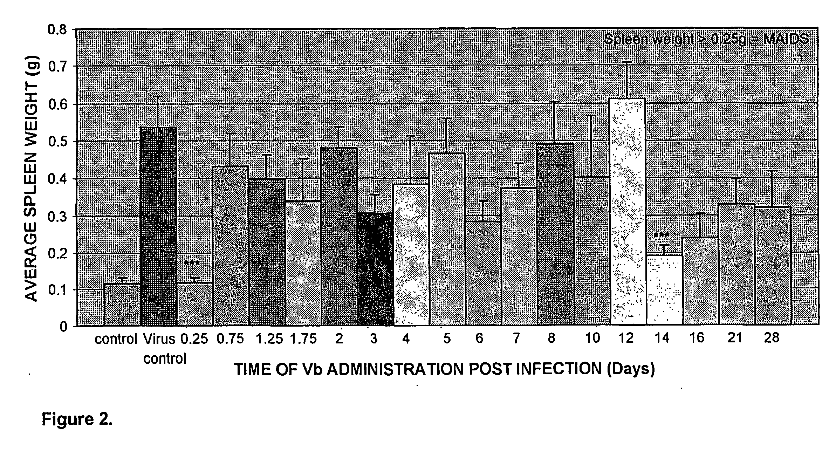

The treatment with a single dose of the anti-mitotic agent vinblastine (Vb) at different times pi is able to prevent the development/progression of MAIDS. Figure 2 shows the effect on MAIDS development (represented by average spleen weight at 10 weeks pi) of a single 6mg/kg i.p. dose of Vb given at various times pi ranging from 6hr to 28 days. Clearly, the Vb treatment is remarkably therapeutic when administered at 6 hr or 14 days pi with 100% (10/10) and 74% (20/27) of mice, respectively, showing no signs of MAIDS development at 10 weeks pi (as determined by spleen weight, histology and FACS analysis). A similar protective effect was observed in some mice treated at 6 days (6/13 or 46%) pi. Although not as pronounced as the protection seen after day 14 treatment, moderate protection from MAINS development was still observed. However, it is also important to note that treatment at many other time points, such as day 2 and day 7 pi, resulted in no protection against MAIDS development. The total protection from MAIDS development seen in mice given Vb at 6 hrs pi is not unexpected as the virus replicates in actively replicating cells which are the target of the anti-mitotic drug thus any cells infected with the virus are killed by the Vb preventing the virus from rapidly establishing an infection in the host. However, the highly protective effect observed after a single treatment with Vb at 14 days pi is quite remarkable as, by this stage, the viral infection is well established and the early disease processes are well underway. We propose that this highly therapeutic effect may be due to the Vb targeting a particular subset of immune regulator cells during their expansion phase and thereby altering the immune response to the viral infection. It is hypothesised that the down-regulation of immune effector cells is removed by the Vb treatment targeting the regulator cells, thus allowing more effective and possibly even complete clearance of the virus by the effector cells of the immune system.

### 2. Further Characterisation of the Day 14 Therapeutic Effect

### 2.1. Spleen histology in Vb treated mice at 10 weeks post infection

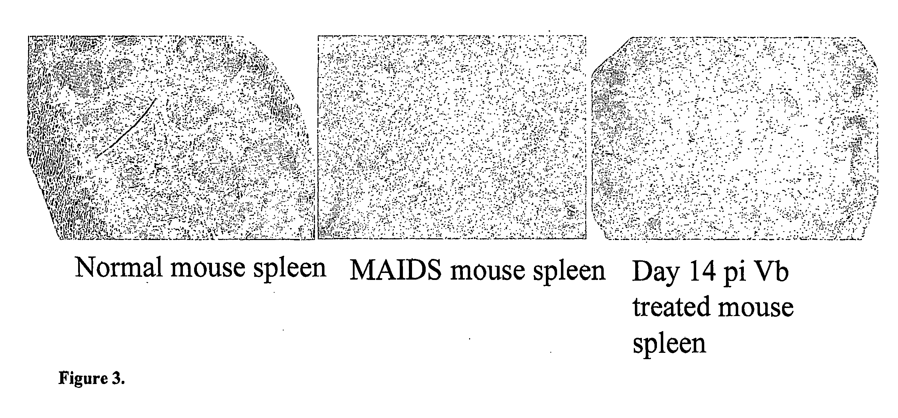

Spleens from the Day 14 pi Vb treated mice as well as infected and uninfected control mice were weighed and examined histologically. The splenic architecture of all MAIDS infected mice was profoundly disorganised as previously reported (Hartley *et al*., 1989). In contrast, the splenic architecture of the Vb treated mice with normal spleen weights (below 0.25g) was indistinguishable from that of uninfected control mice (Figure 3).

In support of this histological data, preliminary results based on FACS analysis of spleen cells from day 14 pi Vb treated mice at 10 weeks pi showed cellular proportions (CD4⁺, CD8⁺ and B cells) and distributions like those observed in normal uninfected mice (data not shown).

### 2.2. Long-term protection from MAIDS development following Vb therapy

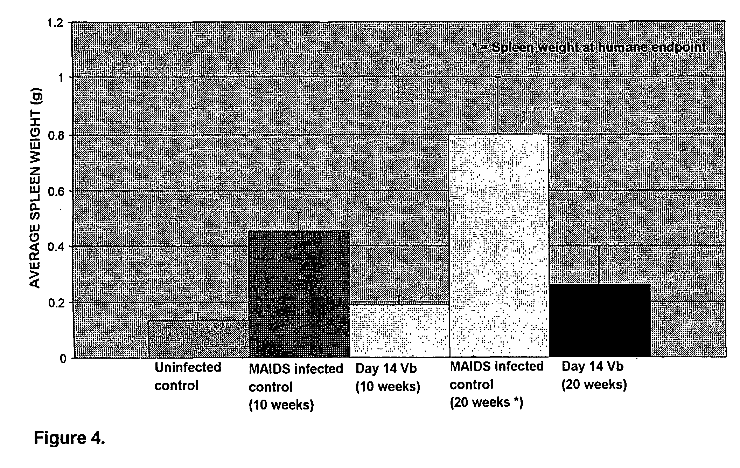

In order to determine if the infected mice were fully protected from MAIDS development or if the Vb treatment was merely delaying the disease onset and progression we treated mice with Vb at day 14 pi and waited until 20 weeks pi to examine the mice. The results of this experiment (Figure 4) showed that 80% (4/5 mice) were protected from any splenomegaly at 20 weeks pi confirming the highly effective action of the day 14 Vb treatment regime.

### 2.3. No protection from virus rechallenge following Vb therapy

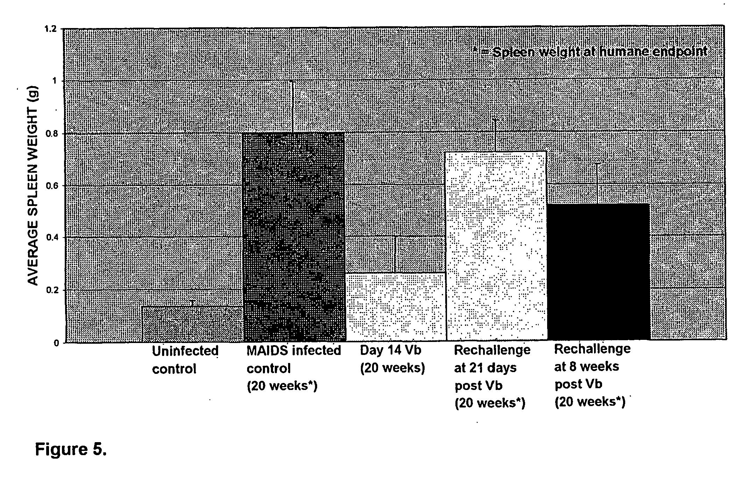

To determine if mice protected from MAIDS by the day 14 pi Vb treatment develop an immune response which would then protect them from a subsequent rechallenge with the MAIDS virus groups of mice, given the Vb therapy at day 14 pi, were rechallenged with the MAIDS virus at 3 or 8 weeks post Vb treatment. The mice were not protected from viral rechallenge and developed splenomegaly and lymphadenopathy indicating MAIDS development at 20 weeks (Figure 5). This is not an unexpected result as the dominant immunosuppression by the regulator population of cells would have been restored as a consequence of the immune effector response to the second (untreated) infection.

### 3. Vb therapy targets cD4+ cells

### 3.1. Direct FACS Analysis of Spleen Cells Following Vb Therapy on Day 14 Post Infection

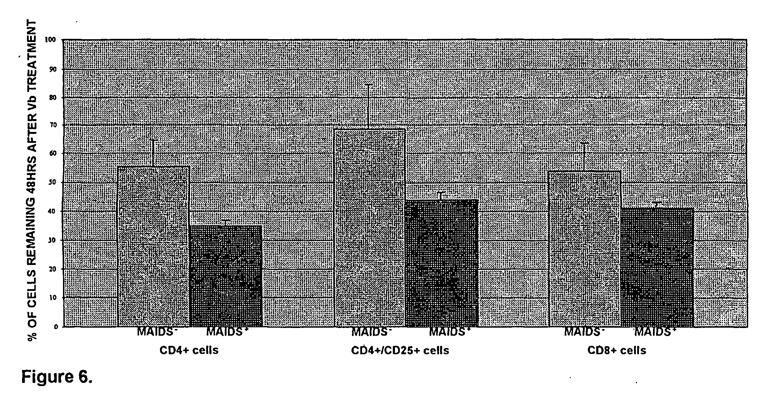

FACS analysis of CD4⁺and CD8⁺ T cells prepared from spleens of mice treated with Vb on Day 14 pi was performed (Figure 6). CD25⁺CD4⁺ T cells were also examined as they have recently been identified as having an important regulatory function in mouse models of autoimmune disease and tumour immunology (Takahashi *et al.,* 1998; Shimizu et al., 2000). It is possible that these cells may play a role in the regulation of the immune response to MAIDS. Day 14 pi and uninfected control mice were given Vb therapy and the spleens collected at 48 hours post Vb treatment. Analysis of the data showed that while all cell subsets are reduced by the Vb therapy, comparison of the uninfected to infected ratios showed that it is the CD4⁺ T cells and CD25⁺CD4⁺ T cells that are preferentially targeted by the Vb treatment in MAIDS infected mice.

### 3.2. Spleen cell transfer experiments: Vb therapy targets CD4⁺ cells

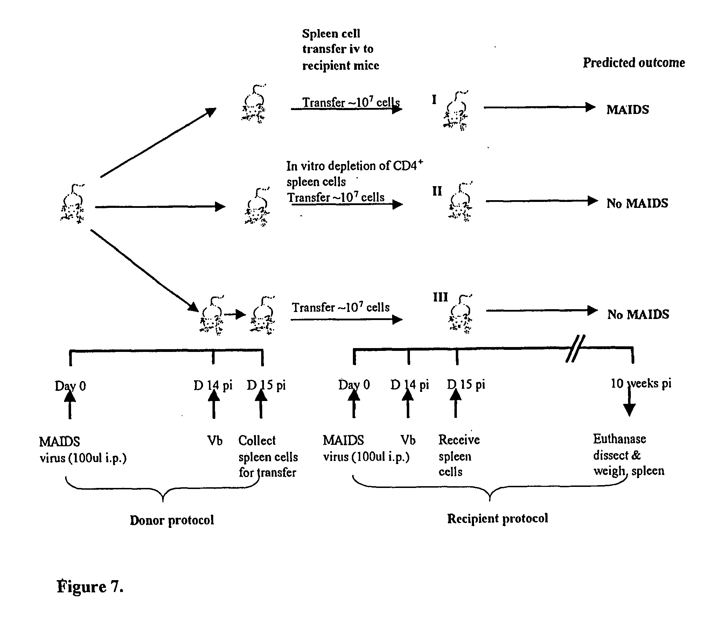

The previous experiment showed that major immune cell subsets (CD4⁺ and CD8⁺ cells) resident in the spleen are all affected by Vb treatment, however, it appears that CD4⁺ cells are preferentially targeted indicating that a subset of these cells may be clonally expanding at day 14 pi. The experiments illustrated in Figure 7 were designed to determine if a single dose of Vb, administered at 14 days pi, is eliminating a population of expanding CD4⁺ regulator T cells and thereby releasing effector cells from down-regulation and clearing the host of the MAIDS virus.

Mice were infected with MAIDS, received day 14 pi Vb treatment and then received an adoptive transfer of ~10⁷ spleen cells from donor mice prepared as outlined in Figure 7. For each experiment [designated (I), (II) and (III)] groups consisted of 7 recipient mice and 9 donor mice. Mice in Group I received whole spleens from infected mice that had not received any Vb treatment. Mice in Group II received whole spleen cell preparations, from infected mice, that had been depleted of CD₄⁺ cells using a FACS cell sorter after staining with fluorochrome-labeled anti-CD4 monoclonal antibody. Cell sorting resulted in removal of 99.5% of CD4⁺ cells from the spleen cell preparations. Mice in Group III received whole spleens from infected donor mice that also received Vb treatment on day 14 pi.

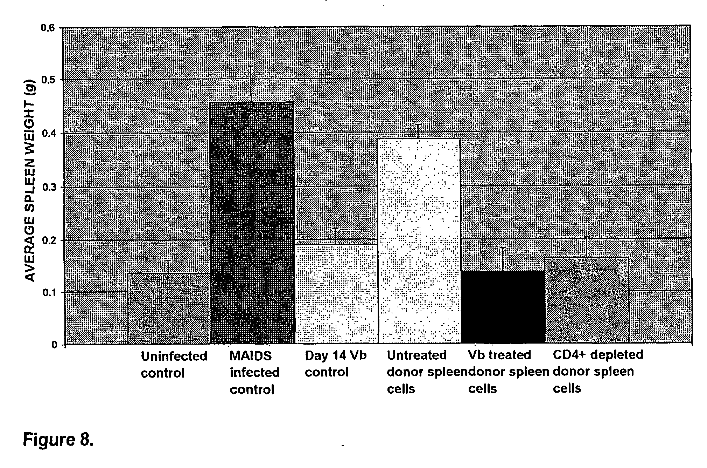

Infusing Vb-treated MAIDS infected mice with spleen cells from MAIDS infected donors completely prevented Vb from protecting mice from MAIDS development (Figure 8). Moreover, the spleen cells that overcame the protective effect of Vb were CD4⁺ cells because when CD4⁺ cells were removed from the donor spleen cells prior to transfer, by FACS sorting, the protective effect of Vb was not overcome. In addition, infusion of Vb-treated MAIDS infected mice with spleen cells from MAIDS infected donor mice that had also been given day 14 Vb showed these mice to be fully protected from MAIDS development (Figure 8).

### 3.3. In vivo depletion of CD4⁺ cells at day 14 post infection results in protection from MAIDS development

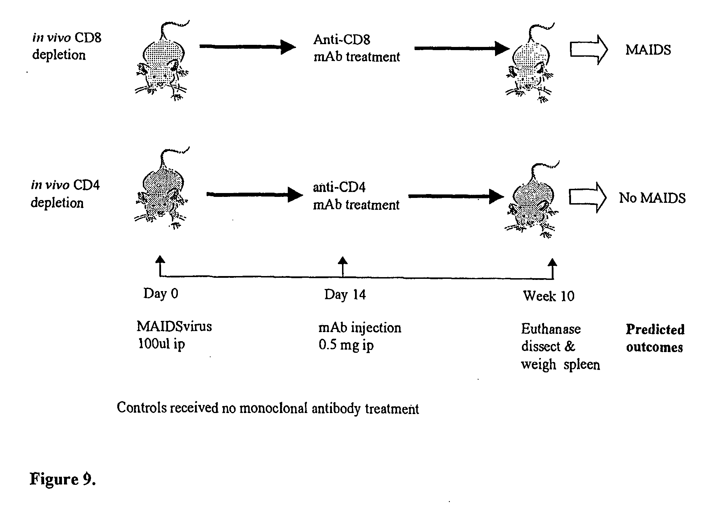

The spleen transfer results are consistent with the interpretation that, at day 14 pi, immune effector cells co-exist with a clonally expanding population of regulator cells which are CD4⁺, and that Vb is therapeutic by virtue of its ability to destroy the latter. Therefore, an *in vivo* depletion of CD4⁺ cells at the 14 day pi time point should mimic the effect of Vb. A flow diagram of the experiment is presented in Figure 9. Groups of 5 mice were infected with the MAIDS virus and then treated with monoclonal antibodies to deplete the host of either the CD4⁺ or CD8⁺ T cell subset at day 14 pi. Monoclonal antibodies were collected from the supernatants of antibody-producing hybridoma cell lines and purified by ammonium sulphate precipitation. Testing *in vivo* resulted in a 98% reduction in CD4⁺ cells and a 95% reduction in CD8⁺ cells from a single injection (0.5mg i.p.) of the appropriate concentrated monoclonal antibody preparation.

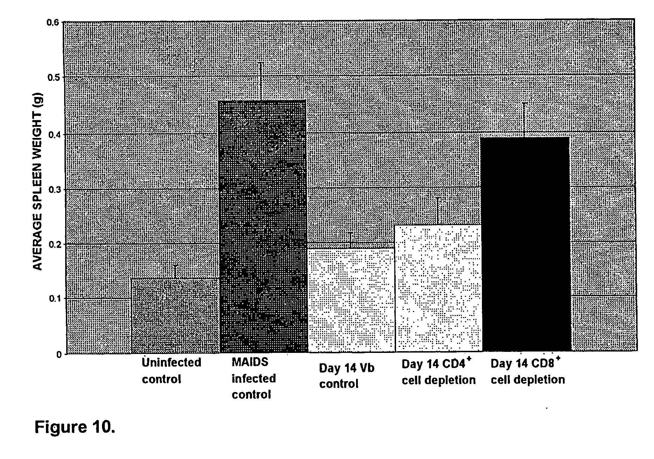

Clearly, treatment at day 14 pi with the anti-CD4⁺ monoclonal antibody resulted in prevention of MAIDS development in infected mice thereby mimicking the effect of a Vb injection at day 14 pi (Figure 10). In contrast, *in vivo* depletion of CD8⁺ cells at day 14 pi had no effect on disease progression. This result further supports the hypothesis that a population of CD4⁺ T cells is functionally down-regulating immune effector cells responding to the viral infection. In addition, the removal of the dominant CD4⁺ regulator cells enables the immune effector cells to respond more effectively to the viral infection resulting in far slower disease progression or perhaps complete clearance of the MAIDS virus from its host.

### 4. Summary

The present examples indicate that there is a population of "regulator" cells that down regulate the immune response to the MAIDS virus, allowing the virus to proliferate and cause disease. Removal of the regulator cells at the appropriate times after infection allows for effector cells (such as B cells and CD8⁺ T cells) to more effectively clear the virus, preventing the development of disease. These regulator cells are proposed to control the activation and/or function of cells such as CD8⁺ T cells and B cells, which act as effector cells, to fight viral infection.

Modulation of the immune response by specifically eliminating a population of immune regulator cells has previously been investigated by Robert North and colleagues using a murine T cell lymphoma model (North and Awwad, 1990). North proposed that the immunogenic tumour was able to establish and grow due to down regulation of the immune response by a population of CD4⁺ regulator T cells. This regulation did not allow the immune response to develop sufficiently in magnitude to cause tumour regression. It was noted that treatment with a single dose of Vinblastine (Vb) at different times following tumour inoculation resulted in enhancement (day 10) or regression (days 4, 6, 13 and 15) of the tumour. North hypothesised that the observed regression was due to elimination of the CD4⁺ regulator T cells at those time points. In a series of experiments involving the selective depletion of CD4⁺ and CD8⁺ T cells the regulator cells were identified as being of the CD4⁺ phenotype.

The chromosomal DNA of inbred and wild mice contains multiple copies of sequences reactive with MuLV nucleic acid probes. Among these sequences are complete, potentially infectious genomes of numerous MuLVs (Chattopadhyay *et al.*, 1980). All MuLVs share high sequence homology. We hypothesize that when these endogenous MuLV proteins are expressed during development they are recognised as self-antigens by the immune system. Due to the high level of homology between endogenous and exogenous MuLV nucleic and amino acid sequences an infecting MuLV may also be partly recognised as self by the immune system, and any immune response to the viral infection will be down-regulated, resulting in a lack of viral clearance and the development of disease.

### REFERENCES:

Barin, F., McLane, M.F., Allan, J.S., Lee, T.H., Groopman, J.E. and Essex, M. (1985). Virus envelope protein of the HTLV-III represents major target antigen for antibodies in AIDS patients. Science 228,1094-6.
Brennan, F.R., Bellaby, T., Helliwell, S.M., Jones, T.D., Kamstrup, S., Dalsgaard, K., Flock, J-I. and Hamilton, W.D.O. (1999). Chimeric plant virus particles administered nasally or orally induce systemic and mucosal immune responses in mice. Journal of Virology 73,930-8.
Coll, J., Palazon, J., Yazbeck, H., Gutierrez, J., Aubo, C., Benito, P., Jagiello, P., Maldyk, H., Marrugat, J. and Anglada, J. et al. (1995). Antibodies to human immunodeficiency virus (HIV-1) in autoimmune diseases: primary Sjogren's syndrome, systemic lupus erythematosus, rheumatoid arthritis and autoimmune thyroid diseases. Clinical Rheumatology 14,451-7.
Cardoso, A.I., Blixenkrone-Moller, M., Fayolle, J., Liu, M., Buckland, R. and Wild, T.F. (1996). Immunization with plasmid DNA encoding for the measles virus hemagglutinin and nucleoprotein leads to humoral and cell-mediated immunity. Virology 225,293-9.
Chattopadhyay, S.K., Lander, M.R., Rands, E. and Lowy, D.R. (1980). Structure of endogenous murine leukemia virus DNA in mouse genomes. Proceedings of the National Academy of Sciences of the USA 77,5774-8.
Conry, R.M., LoBuglio, A.F., Kantor, J., Schlom, J., Loechel, F., Moore, S.E., Sumerel, L.A., Barlow, D.L., Abrams, S. and Curiel, D.T. (1994). Immune response to a carcinoembryonic antigen polynucleotide vaccine. Cancer Research 54,1164-68.
Cox, G.J., Zamb, T.J. and Babiuk, L.A. (1993). Bovine herpesvirus 1: immune responses in mice and cattle injected with plasmid DNA. Journal of Virology 67,5664-7.
Daar, E.S., Bai, J., Hausner, M.A., Majchrowicz, M., Tamaddon, M. and Giorgi, J.V. (1998). Acute HIV syndrome after discontinuation of antiretroviral therapy in a patient treated before seroconversion. Annals of Internal Medicine 128,827-9.
Davis, H.L., Michel, M.L. and Whalen, R.G. (1993). DNA-based immunization induces continuous secretion of hepatitis B surface antigen and high levels of circulating antibody. Human Molecular Genetics 2,1847-51.
Eisenbraun, M.D., Fuller, D.H. and Haynes, J.R. (1993). Examination of parameters affecting the elicitation of humoral immune responses by particle bombardment-mediated genetic immunization. DNA and Cell Biology 12,791-7.
Fynan, E.F., Webster, R.G., Fuller, D.H., Haynes, J.R., Santoro, J.C. and Robinson, H.L. (1993). DNA vaccines: protective immunization by parenteral, mucosal, and gene-gun inoculations. Proceedings of the National Academy of Sciences of the USA 90,11478-82.
Hartley, J.W., Fredrickson, T.N., Yetter, R.A., Makino, M. and Morse, H.C.D. (1989). Retrovirus-induced murine acquired immunodeficiency syndrome: natural history of infection and differing susceptibility of inbred mouse strains. Journal of Virology 63,1223-31.
Hood, E.E. and Jilka, J.M. (1999). Plant-based production of xenogenic proteins. Current Opinions in Biotechnology 10,382-6.
Kapustra, J., Modelska, A., Figlerowicz, M., Pniewski, T., Letellier, M., Lisowa, O., Yusibov, V., Koprowski, H., Plucienniczak, A. and Legocki, A.B. (1999). A plant-derived edible vaccine against hepatitis B virus. FASEB Journal 13,1796-99.
Kilby, J.M., Goepfert, P.A., Miller, A.P., Gnann Jr, J.W., Sillers, M., Saag, M. and Bucy, R.P. (2000). Recurrence of the acute HIV syndrome after interruption of antiretroviral therapy in a patient with chronic HIV infection: A case report. Annals of Internal Medicine 133,435-8.
Lifson, J.D., Rossio, J.L., Arnaout, R., Li, L., Parks, T.L., Schneider, D.K., Kiser, R.F., Coalter, V.J., Walsh, G., Imming, R.J., Fisher, B., Flynn, B.M., Bischofberger, N., Piatak Jr, M., Hirsch, V.M., Nowak, M.A. and Wodarz, D. (2000). Containment of simian immunodeficiency virus infection: cellular immune responses and protection from rechallenge following transient postinoculation antiretroviral treatment. Journal of Virology 74,2584-93.
Mason, H.S., Haq, T., Clements, J. D., and Arntzen, C.J. (1998). Expression of Norwalk virus capsid protein in transgenic tobacco and potato and its oral immunogenicity in mice. Vaccine 16,1336-43.
Mitsuya, H., Yarchoan, R., Kageyama, S. and Broder, S. (1991). Targeted therapy of human immunodeficiency virus-related disease. FASEB Journal 5,2369-81.
Modelska, A., Dietzschold, B., Sleysh, N., Fu, Z.F., Steplewski, K., Hooper, D.C., Koprowski, H. and Yusibov, V. (1998). Immunization against rabies with plant-derived antigen. Proceedings of the National Academy of Sciences of the USA 95,2481-85.
Montgomery, D.L., Shiver, J.W., Leander, K.R., Perry, H.C., Friedman, A., Martinez, D., Ulmer, J.B., Donnelly, J.J. and Lui, M.A. (1993). Heterologous and homologous protection against influenza A by DNA vaccination: optimization of DNA vectors. DNA and Cell Biology 12,777-83.
North, R.J. and Awwad, M. (1990). Elimination of cycling CD4+ suppressor T cells with an anti-mitotic drug releases non-cycling CD8+ T cells to cause regression of an advanced lymphoma. Immunology 71,90-5.
Ortiz, G.M., Nixon, D.F., Trkola, A., Binley, J., Jin, X., Bonhoeffer, S., Kuebler, P.J., Donahoe, S.M., Demoitie, M.-A., Kakimoto, W.M., Ketas, T., et al. (1999). HIV-1-specific immune responses in subjects who temporarily contain virus replication after discontinuation of highly active antiretroviral therapy. Journal of Clinical Investigation, published online, September 1999.
Rakowicz-Szulczynska, E.M. (2000). Relevance of the viral RAK alpha gene in diagnosis of malignant versus nonmalignant tumors of the ovary and uterus. Clinical and Diagnostic Laboratory Immunology 7,360-5.
Rakowicz-Szulczynska, E.M., Jackson, B., Szulczynska, A.M. and Smith, M. (1998). Human immunodeficieny virus type 1-like DNA sequences and immunoreactive viral particles with unique association with breast cancer. Clinical and Diagnostic Laboratory Immunology 5,645-53.
Sedegah, M., Hedstrom, R., Hobart, P. and Hoffman, S.L. (1994). Protection against malaria by immunization with plasmid DNA encoding circumsporozoite protein. Proceedings of the National Academy of Sciences of the USA 91,9866-70.
Shimizu, J., Yamazaki, S. and Sakaguchi, S. (1999). Induction of tumor immunity by removing CD25+CD4+ T cells: a common basis between tumor immunity and autoimmunity. Journal of Immunology 163,5211-8.
Stott, J., Hu, S.L., and Almond, N. (1998). Candidate vaccines protect macaques against primate immunodeficiency viruses. AIDS Research and Human Retroviruses. Suppl 3,S265-70.
Takahashi, T., Kuniyasu, Y., Toda, M., Sakaguchi, N., Itoh, M., Iwata, M., Shimizu, J. and Sakaguchi, S. (1998). Immunologic self-tolerance maintained by CD25+CD4+ naturally anergic and suppressive T cells: induction of autoimmune disease by breaking their anergic/suppressive state. International Immunology 10,1969-80.
Ulmer, J.B., Donnelly, J.J., Parker, S.E., Rhodes, G.H., Felgner, P.L., Dwarki, V.J., Gromkowski, S.H., Deck, R.R., DeWitt, C.M., Friedman, A. et al. (1993). Heterologous protection against influenza by injection of DNA encoding a viral protein. Science 259,1745-49.
Wang, B., Ugen, K.E., Srikantan, V., Agadjanyan, M.G., Dang, K., Refaeli, Y., Sato, A.I., Boyer, J., Williams, W.V. and Weiner, D.B. (1993). Gene inoculation generates immune responses against human immunodeficiency virus type 1. Proceedings of the National Academy of Sciences of the USA 90,4156-60.
Xiang, Z.Q., Spitalnik, S., Tran, M., Wunner, W.H., Cheng, J. and Ertl, H.C. (1994). Vaccination with a plasmid vector carrying the rabies virus glycoprotein gene induces protective immunity against rabies virus. Virology 199,132-40.
Yang, K., Mustafa, F., Valsamakis, A., Santoro, J.C., Griffin, D.E. and Robinson, H.L. (1997). Early studies on DNA-based immunization for measles virus. Vaccine 15,888-91.

## Claims

1. Use of a composition comprising a retroviral antigenic polypeptide or DNA encoding the polypeptide for the manufacture of a medicament for administering to a mammalian subject with a retroviral infection, wherein the subject is subsequently administered with an agent which inhibits the production of, and/or destroys, regulator T cells, and wherein the timing of administration of the agent is selected such that it exerts a proportionally greater effect against the regulator T cells than the effector T cells, and wherein the agent is selected from the group consisting of anti-proliferative drugs, radiation and an antibody which binds specifically to CD4+ T cells.

2. Use of an agent which inhibits the production of, and/or destroys, regulator T cells for the manufacture of a medicament for administering to a mammalian subject with a retroviral infection, wherein the subject has previously been administered with a composition comprising a retroviral antigenic polypeptide or DNA encoding the polypeptide, and wherein the agent is administered at a time selected such that it exerts a proportionally greater effect against the regulator T cells than the effector T cells, and wherein the agent is selected from the group consisting of anti-proliferative drugs, radiation and an antibody which binds specifically to CD4+ T cells.

3. Use of a composition comprising a retroviral antigenic polypeptide or DNA encoding the polypeptide, and an agent which inhibits the production of, and/or destroys, regulator T cells for the manufacture of a medicament for administering to a mammalian subject with a retroviral infection, wherein the subject is administered with the composition before the agent, and wherein the timing of administration of the agent is selected such that it exerts a proportionally greater effect against the regulator T cells than the effector T cells, and wherein the agent is selected from the group consisting of anti-proliferative drugs, radiation and an antibody which binds specifically to CD4+ T cells.

4. The use according to any one of claims 1 to 3, wherein the agent is administered approximately when CD8+CD4- T cell numbers have peaked in response to the administration of the composition.

5. The use according to any one of claims 1 to 3, wherein the agent is administered approximately when the number of viral particles has begun to stablize or increase following administration of the composition.

6. The use according to any one of claims 1 to 5, wherein the retroviral antigenic polypeptide is provided to the subject by administering a vaccine comprising the retrovirus antigenic polypeptide and a pharmaceutically acceptable carrier.

7. The use of claim 6, wherein the vaccine further comprises an adjuvant.

8. The use according to any one of claims 1 to 5, wherein the antigenic polypeptide is provided to the subject by administering a DNA vaccine encoding the retroviral antigenic polypeptide.

9. The use according to any one of claims 1 to 5, wherein the antigenic polypeptide is provided to the subject by the consumption of a transgenic plant expressing the retroviral antigenic polypeptide.

10. The use according to any one of claims 1 to 9, wherein the subject has been exposed to antiretroviral drug therapy before the composition is administered.

11. Use of an antiretroviral drug for the manufacture of a medicament for administering to a mammalian subject with a retroviral infection, wherein the subject is exposed to antiretroviral drug therapy, and subsequently administered with an agent which inhibits the production of, and/or destroys, regulator T cells, wherein the agent is administered after the antiretroviral drug therapy has concluded and a resulting expansion in retroviral numbers has led to an increase in the number, and/or activation, of effector T cells directed against the retrovirus, and wherein the timing of administration of the agent is selected such that it exerts a proportionally greater effect against the regulator T cells than the effector T cells, and wherein the agent is selected from the group consisting of anti-proliferative drugs, radiation and an antibody which binds specifically to CD4+ T cells.

12. Use of an agent which inhibits the production of, and/or destroys, regulator T cells for the manufacture of a medicament for administering to a mammalian subject with a retroviral infection, wherein the subject has been exposed to antiretroviral drug therapy, and wherein the agent is administered after the antiretroviral drug therapy has concluded and a resulting expansion in retroviral numbers has led to an increase in the number, and/or activation, of effector T cells directed against the retrovirus, and wherein the timing of administration of the agent is selected such that it exerts a proportionally greater effect against the regulator T cells than the effector T cells, and wherein the agent is selected from the group consisting of anti-proliferative drugs, radiation and an antibody which binds specifically to CD4+ T cells.

13. Use of an antiretroviral drug, and an agent which inhibits the production of, and/or destroys, regulator T cells for the manufacture of a medicament for administering to a mammalian subject with a retroviral infection, wherein the subject is exposed antiretroviral drug therapy, and subsequently administered with the agent, and wherein the agent is administered after the antiretroviral drug therapy has concluded and a resulting expansion in retroviral numbers has led to an increase in the number, and/or activation, of effector T cells directed against the retrovirus, and wherein the timing of administration of the agent is selected such that it exerts a proportionally greater effect against the regulator T cells than the effector T cells, and wherein the agent is selected from the group consisting of anti-proliferative drugs, radiation and an antibody which binds specifically to CD4+ T cells.

14. The use according to any one of claims 11 to 13, wherein the agent is administered approximately when CD8+CD4- T cell numbers have peaked in response to the conclusion of the antiretroviral drug therapy.

15. The use according to any one of claims 11 to 13, wherein the agent is administered approximately when the number of viral particles has peaked, or begun to decrease after this peak, in response to the conclusion of the antiretroviral drug therapy.

16. The use according to any one of claims 1 to 15, wherein the anti-proliferative drug is selected from the group consisting of vinblastine and anhydro vinblastine.

17. The use of according to any one of claims 1 to 16, wherein the retrovirus is selected from the group consisting of HTV-1, HIV-2, HTLV-1 and HTLV-2.

18. The use according to any one of claims 1 to 17, wherein the method is repeated at least once.

19. The use according to any one of claims 1 to 18, wherein the mammalian subject is a human.

20. The use according to any one of claims 1 to 19 wherein the anti-proliferative drug is selected from mechlorethamine, cyclophosphamide, ifosfamide, melphalan, chlorambucil, hexamethyl-melamine, thiotepa, busulfan, carmustine, lomustine, semustine, streptozocin, dacarbazine, methotrexate, fluorouracil, floxuridine, cytarabine, mercaptopurine, thioguanine, pentostatin, vinblastine, anhydro vinblastine, vincristine, etoposide, teniposide, dactinomycin, daunorubicin, doxorubicin, bleomycin, plicamycin, mitomycin, L-asparaginase, cisplatin, mitoxantrone, procarbazine, mitotane, aminoglutethimide, prednisone, hydroxyprogesterone caproate, medroprogesterone acetate, megestrol acetate, diethylstilbestrol, ethinyl estradiol, tamoxifen, testosterone propionate, radioactive isotopes, ricin A chain, taxol, diphtheria toxin and pseudomonas exotoxin A.

21. A method for determining when an agent which inhibits the production of, and/or destroys, regulator T cells, is to be administered to a mammalian subject with a retroviral infection that has been exposed to a treatment which increases the number of, and/or activates, effector T cells directed against the retrovirus, the method comprising (i) analysing a sample previously obtained from the subject for (a) effector T cell numbers and/or (b) regulator T cell numbers or activity, or a marker thereof, or (ii) monitoring viral load in the subject; wherein the agent is selected from the group consisting of anti-proliferative drugs, radiation and an antibody which binds specifically to CD4+ T cells.

22. The method of claim 21, wherein the method includes determining CD8+CD4- T cell numbers.

23. The method of claim 21, wherein the method includes determining CD4+CD8- T cell numbers.

24. The method of claim 21, wherein the method includes determining the number of viral particles,

## Patentansprüche

1. Verwendung einer Zusammensetzung, die ein retrovirales antigenes Polypeptid oder eine das Polypeptid kodierende DNA umfasst, für die Herstellung eines Medikaments zur Verabreichung an ein Säugetier-Subjekt mit einer retroviralen Infektion, wobei dem Subjekt anschließend ein Mittel verabreicht wird, das die Herstellung von regulatorischen T-Zellen hemmt und/oder regulatorische T-Zellen zerstört, wobei die Zeitabstimmung der Verabreichung des Mittels so gewählt wird, dass es einen proportional größeren Effekt gegen die regulatorischen T-Zellen ausübt als Effektor-T-Zellen, wobei das Mittel ausgewählt ist aus der Gruppe, bestehend aus antiproliferativen Arzneimitteln, Bestrahlung und einem Antikörper, der spezifisch an CD4+ T-Zellen bindet.

2. Verwendung eines Mittels, das die Herstellung von regulatorischen T-Zellen hemmt und/oder regulatorische T-Zellen zerstört, für die Herstellung eines Medikaments zur Verabreichung an ein Säugetier-Subjekt mit einer retroviralen Infektion, wobei dem Subjekt zuvor eine Zusammensetzung verabreicht wurde, die ein retrovirales antigenes Polypeptid oder das Polypeptid kodierende DNA umfasst, wobei das Mittel zur einem Zeitpunkt verabreicht wird, der so ausgewählt ist, dass es einen proportional größeren Effekt gegen die regulatorischen T-Zellen ausübt als die Effektor-T-Zellen, und wobei das Mittel ausgewählt ist aus der Gruppe, bestehend aus antiproliferativen Arzneimitteln, Bestrahlung und einem Antikörper, der spezifisch an CD4+ T-Zellen bindet.

3. Verwendung einer Zusammensetzung, die ein retrovirales antigenes Polypeptid oder das Polypeptid kodierende DNA umfasst, und eines Mittels, das die Herstellung von regulatorischen T-Zellen hemmt und/oder regulatorische T-Zellen zerstört, für die Herstellung eines Medikamentes zur Verabreichung an ein Säugetier-Subjekt mit einer retroviralen Infektion, wobei dem Subjekt die Zusammensetzung vor dem Mittel verabreicht wird, und wobei die Zeitabstimmung der Verabreichung des Mittels so gewählt ist, dass es einen proportional größeren Effekt gegen die regulatorischen T-Zellen ausübt als die Effektor-T-Zellen, und wobei das Mittel ausgewählt ist aus der Gruppe, bestehend aus antiproliferativen Arzneimitteln, Bestrahlung und einem Antikörper, der spezifisch an CD4+ T-Zellen bindet.

4. Verwendung gemäß irgendeinem der Ansprüche 1 bis 3, wobei das Mittel ungefähr dann verabreicht wird, wenn die Anzahlen der CD8+CD4- T-Zellen als Antwort auf die Verabreichung der Zusammensetzung ihren Höhepunkt erreichen.

5. Verwendung gemäß irgendeinem der Ansprüche 1 bis 3, wobei das Mittel ungefähr dann verabreicht wird, wenn die Anzahl der viralen Partikel nach Verabreichung der Zusammensetzung sich stabilisiert oder ansteigt.

6. Verwendung gemäß irgendeinem der Ansprüche 1 bis 5, wobei das retrovirale antigene Polypeptid dem Subjekt durch Verabreichung eines Impfstoffs bereitgestellt wird, der das antigene Retrovirus-Polypeptid und einen pharmazeutisch verträglichen Träger umfasst.

7. Verwendung nach Anspruch 6, wobei der Impfstoff weiter einen Hilfsstoff umfasst.

8. Verwendung gemäß irgendeinem der Ansprüche 1 bis 5, wobei das antigene Polypeptid dem Subjekt durch Verabreichung eines DNA-Impfststoffs bereitgestellt wird, der das retrovirale antigene Polypeptid kodiert.

9. Verwendung gemäß irgendeinem der Ansprüche 1 bis 5, wobei das antigene Polypeptid dem Subjekt durch Verbrauch einer transgenen Pflanze bereitgestellt wird, die das retrovirale antigene Polypeptid exprimiert.

10. Verwendung gemäß irgendeinem der Ansprüche 1 bis 9, wobei das Subjekt einer antiretroviralen Arzneimitteltherapie ausgesetzt worden war, bevor die Zusammensetzung verabreicht wird.

11. Verwendung eines antiretroviralen Arzneimittels für die Herstellung eines Medikaments zur Verabreichung an ein Säugetier-Subjekt mit einer retroviralen Infektion, wobei das Subjekt einer antiretroviralen Arzneimitteltherapie ausgesetzt wird und ihm anschließend ein Mittel verabreicht wird, das die Herstellung von regulatorischen T-Zellen hemmt und/oder regulatorische T-Zellen zerstört, wobei das Mittel verabreicht wird, nachdem die antiretrovirale Arzneimitteltherapie abgeschlossen wurde und ein daraus folgendes Ansteigen retroviraler Anzahlen zu einem Ansteigen der Anzahl und/oder Aktivierung von Effektor-T-Zellen geführt hat, die gegen das Retrovirus gerichtet sind, und wobei die Zeitabstimmung der Verabreichung des Mittels so gewählt wird, dass es einen proportional größeren Effekt gegen die regulatorischen T-Zellen ausübt als die Effektor-T-Zellen, und wobei das Mittel ausgewählt ist aus der Gruppe, bestehend aus antiproliferativen Arzneimitteln, Bestrahlung und einem Antikörper, der spezifisch an CD4+ T-Zellen bindet.

12. Verwendung eines Mittels, das die Herstellung von regulatorischen T-Zellen hemmt und/oder regulatorische T-Zellen zerstört, für die Herstellung eines Medikaments zur Verabreichung an ein Säugetier-Subjekt mit einer retroviralen Infektion, wobei das Subjekt einer antiretroviralen Arzneimitteltherapie ausgesetzt worden war, und wobei das Mittel verabreicht wird, nachdem die antiretrovirale Arzneimitteltherapie abgeschlossen wurde und ein daraus folgendes Ansteigen retroviraler Anzahlen zu einem Ansteigen der Anzahl und/oder Aktivierung von Effektor-T-Zellen geführt hat, die gegen das Retrovirus gerichtet sind, und wobei die Zeitabstimmung der Verabreichung des Mittels so gewählt wird, dass es einen proportional größeren Effekt gegen die regulatorischen T-Zellen ausübt als die Effektor-T-Zellen, und wobei das Mittel ausgewählt ist aus der Gruppe, bestehend aus antiproliferativen Arzneimitteln, Bestrahlung und einem Antikörper, der spezifisch an CD4+ T-Zellen bindet.

13. Verwendung eines antiretroviralen Arzneimittels und eines Mittels, das die Herstellung von regulatorischen T-Zellen hemmt und/oder regulatorische T-Zellen zerstört, für die Herstellung eines Medikaments zur Verabreichung an ein Säugetier-Subjekt mit einer retroviralen Infektion, wobei das Subjekt einer antiretroviralen Arzneimitteltherapie ausgesetzt wird und ihm anschließend das Mittel verabreicht wird, und wobei das Mittel verabreicht wird, nachdem die antiretrovirale Arzneimitteltherapie abgeschlossen wurde und ein daraus folgendes Ansteigen retroviraler Anzahlen zu einem Ansteigen der Anzahl und/oder Aktivierung von Effektor-T-Zellen geführt hat, die gegen das Retrovirus gerichtet sind, und wobei die Zeitabstimmung der Verabreichung des Mittels so gewählt wird, dass es einen proportional größeren Effekt gegen die regulatorischen T-Zellen ausübt als die Effektor-T-Zellen, und wobei das Mittel ausgewählt ist aus der Gruppe, bestehend aus antiproliferativen Wirkstoffen, Bestrahlung und einem Antikörper, der spezifisch an CD4+ T-Zellen bindet.

14. Verwendung gemäß irgendeinem der Ansprüche 11 bis 13, wobei das Mittel ungefähr dann verabreicht wird, wenn die Anzahlen an CD8+CD4- T-Zellen als Antwort auf das Abschließen der antiretroviralen Arzneimitteltherapie ihren Höhepunkt erreicht haben.

15. Verwendung gemäß irgendeinem der Ansprüche 11 bis 13, wobei das Mittel ungefähr dann verabreicht wird, wenn die Anzahl der viralen Partikel als Antwort auf das Abschließen der antiretroviralen Arzneimitteltherapie ihren Höhepunkt erreicht hat oder nach diesem Höhepunkt begonnen hat, kleiner zu werden.

16. Verwendung gemäß irgendeinem der Ansprüche 1 bis 15, wobei das antiproliferative Arzneimittel ausgewählt ist aus der Gruppe, bestehend aus Vinblastin und Anhydrovinblastin.

17. Verwendung gemäß irgendeinem der Ansprüche 1 bis 16, wobei das Retrovirus ausgewählt ist aus der Gruppe, bestehend aus HIV-1, HIV-2, HTLV-1 und HTLV-2.

18. Verwendung gemäß irgendeinem der Ansprüche 1 bis 17, wobei das Verfahren wenigstens einmal wiederholt wird.

19. Verwendung gemäß irgendeinem der Ansprüche 1 bis 18, wobei das Säugetier-Subject ein Mensch ist.

20. Verwendung gemäß irgendeinem der Ansprüche 1 bis 19, wobei das antiproliferative Arzneimittel ausgewählt ist unter Mechlorethamin, Cyclophosphamid, Ifosfamid, Melphalan, Chlorambucil, Hexamethylmelamin, Thiotepa, Busulfan, Carmustin, Lomustin, Semustin, Streptozocin, Dacarbazin, Methotrexat, Fluoruracil, Floxuridin, Cytarabin, Mercaptopurin, Thioguanin, Pentostatin, Vinblastin, Anhydrovinblastin, Vincristin, Etoposid, Teniposid, Dactinomycin, Daunorubicin, Doxorubicin, Bleomycin, Plicamycin, Mitomycin, L-Asparaginase, Cisplatin, Mitoxantron, Procarbazin, Mitotan, Aminoglutethimid, Prednison, Hydroxyprogesteroncaproat, Medroprogesteronacetat, Megestrolacetat, Diethylstilbestrol, Ethinylestradiol, Tamoxifen, Testosteronpropionat, radioaktiven Isotopen, Ricin A-Kette, Taxol, Diphtherietoxin, Pseudomonas Exotoxin A.

21. Verfahren zur Bestimmung, wann ein Mittel, das die Herstellung von regulatorischen T-Zellen hemmt und/oder regulatorische T-Zellen zerstört, einem Säugetier-Subjekt mit einer retroviralen Infektion verabreicht werden soll, das einer Behandlung ausgesetzt war, die die Anzahl von Effektor-T-Zellen erhöht und/oder Effektor-T-Zellen aktiviert, die gegen das Retrovirus gerichtet sind, wobei das Verfahren umfasst: (i) Analysieren einer Probe, die zuvor von dem Subjekt erhalten wurde nach (a) Anzahlen von Effektor-T-Zellen und/oder (b) Anzahlen oder Aktivität von regulatorischen T-Zellen oder einem Marker davon, oder (ii) Überwachen der viralen Belastung des Subjekts, wobei das Mittel ausgewählt ist aus der Gruppe, bestehend aus antiproliferativen Arzneimitteln, Bestrahlung und einem Antikörper, der spezifisch an CD4+ T-Zellen bindet.

22. Verfahren nach Anspruch 21, wobei das Verfahren das Bestimmen von Anzahlen von CD8+CD4- T-Zellen umfasst.

23. Verfahren nach Anspruch 21, wobei das Verfahren das Bestimmen von Anzahlen von CD4+CD8- T-Zellen umfasst.

24. Verfahren nach Anspruch 21, wobei das Verfahren das Bestimmen der Anzahl von viralen Partikeln umfasst.

## Revendications

1. Utilisation d'une composition comprenant un polypeptide antigénique rétroviral ou un ADN codant pour le polypeptide pour la fabrication d'un médicament à administrer à un sujet mammifère ayant une infection rétrovirale, où on administre ensuite au sujet un agent qui inhibe la production des lymphocytes T régulateurs et/ou les détruit, et où le moment de l'administration de l'agent est choisi de façon qu'il exerce un effet proportionnellement plus grand contre les lymphocytes T régulateurs que contre les lymphocytes T effecteurs, et où l'agent est choisi dans le groupe constitué par des médicaments antiprolifératifs, des rayonnements et un anticorps qui se lie de façon spécifique aux lymphocytes T CD4+.

2. Utilisation d'un agent qui inhibe la production des lymphocytes T régulateurs et/ou les détruit pour la fabrication d'un médicament à administrer à un sujet mammifère ayant une infection rétrovirale, où le sujet a auparavant reçu une composition comprenant un polypeptide antigénique rétroviral ou un ADN codant pour le polypeptide, et l'agent est administré à un moment choisi de façon qu'il exerce un effet proportionnellement plus grand contre les lymphocytes T régulateurs que contre les lymphocytes T effecteurs, et où l'agent est choisi dans le groupe constitué par des médicaments antiprolifératifs, des rayonnements et un anticorps qui se lie de façon spécifique aux lymphocytes T CD4+.

3. Utilisation d'une composition comprenant un polypeptide antigénique rétroviral ou un ADN codant pour le polypeptide, et d'un agent qui inhibe la production des lymphocytes T régulateurs et/ou les détruit, pour la fabrication d'un médicament à administrer à un sujet mammifère ayant une infection rétrovirale, où on administre au sujet la composition avant l'agent, et où le moment de l'administration de l'agent est choisi de façon qu'il exerce un effet proportionnellement plus grand contre les lymphocytes T régulateurs que contre les lymphocytes T effecteurs, et où l'agent est choisi dans le groupe constitué par des médicaments antiprolifératifs, des rayonnements et un anticorps qui se lie de façon spécifique aux lymphocytes T CD4+.

4. Utilisation selon l'une quelconque des revendications 1 à 3, où l'agent est administré à peu près lorsque les nombres de lymphocytes T CD8+CD4- ont atteint un maximum en réponse à l'administration de la composition.

5. Utilisation selon l'une quelconque des revendications 1 à 3, où l'agent est administré approximativement lorsque le nombre de particules virales a commencé à se stabiliser ou à augmenter après l'administration de la composition.

6. Utilisation selon l'une quelconque des revendications 1 à 5, où le polypeptide antigénique rétroviral est donné au sujet par administration d'un vaccin comprenant le polypeptide antigénique rétroviral et un support pharmaceutiquement acceptable.

7. Utilisation selon la revendication 6, où le vaccin comprend en outre un adjuvant.

8. Utilisation selon l'une quelconque des revendications 1 à 5, où le polypeptide antigénique est donné au sujet par administration d'un vaccin à ADN codant pour le polypeptide antigénique rétroviral.

9. Utilisation selon l'une quelconque des revendications 1 à 5, où le polypeptide antigénique est donné au sujet par consommation d'une plante transgénique exprimant le polypeptide antigénique rétroviral.

10. Utilisation selon l'une quelconque des revendications 1 à 9, où le sujet a été exposé à une thérapie avec un médicament antirétroviral avant l'administration de la composition.

11. Utilisation d'un médicament antirétroviral pour la fabrication d'un médicament à administrer à un sujet mammifère ayant une infection rétro virale, où le sujet est exposé à une thérapie avec un médicament antirétroviral, puis reçoit un agent qui inhibe la production des lymphocytes T régulateurs et/ou les détruit, et où l'agent est administré après que la thérapie avec le médicament antirétroviral s'est achevée et que l'accroissement des nombres rétroviraux qui en résulte a entraîné une augmentation du nombre et/ou l'activation des lymphocytes T effecteurs dirigés contre le rétrovirus, et où le moment de l'administration de l'agent est choisi de façon qu'il exerce un effet proportionnellement plus grand contre les lymphocytes T régulateurs que contre les lymphocytes T effecteurs, et où l'agent est choisi dans le groupe constitué par des médicaments antiprolifératifs, des rayonnements et un anticorps qui se lie de façon spécifique aux lymphocytes T CD4+.

12. Utilisation d'un agent qui inhibe la production de lymphocytes T régulateurs et/ou les détruit pour la fabrication d'un médicament à administrer à un sujet mammifère ayant une infection rétrovirale, où le sujet a été exposé à une thérapie avec un médicament antirétroviral, et où l'agent est administré après que la thérapie avec le médicament antirétroviral s'est achevée et que l'expansion des nombres rétroviraux qui en résulte a entraîné une augmentation du nombre et/ou l'activation des lymphocytes T effecteurs dirigés contre le rétrovirus, et où le moment de l'administration de l'agent est choisi de façon qu'il exerce un effet proportionnellement plus grand contre les lymphocytes T régulateurs que contre les lymphocytes T effecteurs, et où l'agent est choisi dans le groupe constitué par des médicaments antiprolifératifs, des rayonnements et un anticorps qui se lie de façon spécifique aux lymphocytes T CD4+.

13. Utilisation d'un médicament antirétroviral et d'un agent qui inhibe la production de lymphocytes T régulateurs et/ou les détruit pour la fabrication d'un médicament à administrer à un sujet mammifère ayant une infection rétrovirale, où le sujet est exposé à une thérapie avec un médicament antirétroviral et reçoit ensuite l'agent, et où l'agent est administré après que la thérapie avec le médicament antirétroviral s'est achevée et que l'expansion des nombres rétroviraux qui en résulte a entraîné une augmentation du nombre et/ou l'activation des lymphocytes T effecteurs dirigés contre le rétrovirus, et où le moment de l'administration de l'agent est choisi de façon qu'il exerce un effet proportionnellement plus grand contre les lymphocytes T régulateurs que contre les lymphocytes T effecteurs, et où l'agent est choisi dans le groupe constitué par des médicaments antiprolifératifs, des rayonnements et un anticorps qui se lie de façon spécifique aux lymphocytes T CD4+.

14. Utilisation selon l'une quelconque des revendications 11 à 13, où l'agent est administré à peu près lorsque les nombres de lymphocytes T CD8+CD4- ont atteint un maximum en réponse à l'achèvement de la thérapie avec le médicament antirétroviral.

15. Utilisation selon l'une quelconque des revendications 11 à 13, où l'agent est administré à peu près lorsque le nombre de particules virales a atteint un maximum ou a commencé à baisser après ce maximum, en réponse à l'achèvement de la thérapie avec le médicament antirétroviral.

16. Utilisation selon l'une quelconque des revendications 1 à 15, où le médicament antiprolifératif est choisi dans le groupe constitué par la vinblastine et l'anhydrovinblastine.

17. Utilisation selon l'une quelconque des revendications 1 à 16, où le rétrovirus est choisi dans le groupe constitué par le VIH-1, le VIH-2, le HTLV-1 et le HTLV-2.

18. Utilisation selon l'une quelconque des revendications 1 à 17, où le procédé est répété au moins une fois.

19. Utilisation selon l'une quelconque des revendications 1 à 18, où le sujet mammifère est un être humain.

20. Utilisation selon l'une quelconque des revendications 1 à 19, où le médicament antiprolifératif est choisi parmi la méchloréthamine, le cyclophosphamide, l'ifosfamide, le melphalan, le chlorambucil, l'hexaméthyl-mélamine, le thiotépa, le busulfan, la carmustine, la lomustine, la sémustine, la streptozocine, la dacarbazine, le méthotrexate, le fluorouracile, la floxuridine, la cytarabine, la mercaptopurine, la thioguanine, la pentostatine, la vinblastine, l'anhydrovinblastine, la vincristine, l'étoposide, le téniposide, la dactinomycine, la daunorubicine, la doxorubucine, la bléomycine, la plicamycine, la mitomycine, la L-asparaginase, le cisplatine, la mitoxantrone, la procarbazine, le mitotane, l'aminoglutéthimide, la prednisone, le caproate d'hydroxyprogestérone, l'acétate de médroprogestérone, l'acétate de mégestrol, le diéthylstilbestrol, l'éthynylestradiol, le tamoxifène, le propionate de testostérone, des isotopes radioactifs, la chaîne A de la ricine, le taxol, la toxine diphtérique et l'exotoxine A de Pseudomonas.

21. Procédé pour déterminer le moment où il faut administrer un agent qui inhibe la production de lymphocytes T régulateurs et/ou les détruit à un sujet mammifère ayant une infection rétrovirale et qui a été exposé à un traitement qui augmente le nombre de lymphocytes T effecteurs dirigés contre le rétrovirus et/ou active ces lymphocytes T effecteurs, le procédé comprenant (i) l'analyse d'un échantillon préalablement prélevé chez le sujet pour obtenir (a) le nombre des lymphocytes T effecteurs et/ou (b) le nombre ou l'activité des lymphocytes T régulateurs, ou un marqueur de ceux-ci, ou (ii) le suivi de la charge virale chez le sujet; où l'agent est choisi dans le groupe constitué par des médicaments antiprolifératifs, des rayonnements et un anticorps qui se lie de façon spécifique aux lymphocytes T CD4+.

22. Procédé selon la revendication 21, le procédé comprenant la détermination du nombre de lymphocytes T CD8+CD4-.

23. Procédé selon la revendication 21, le procédé comprenant la détermination du nombre de lymphocytes T CD4+CD8-.

24. Procédé selon la revendication 21, le procédé comprenant la détermination du nombre de particules virales.
